(19) Europäisches Patentamt
European Patent Office
Office européen des brevets

(11) Numéro de publication : **0 087 347**
**B1**

(12) # FASCICULE DE BREVET EUROPÉEN

(45) Date de publication du fascicule du brevet : **22.01.86**

(51) Int. Cl.$^4$ : **C 07 D263/58**

(21) Numéro de dépôt : **83400269.3**

(22) Date de dépôt : **08.02.83**

(54) **Procédé de préparation de benzoxazolone-2 et dérivés à partir d'orthonitrophénols et de monoxyde de carbone.**

(30) Priorité : **23.02.82 FR 8202921**

(43) Date de publication de la demande :
**31.08.83 *Bulletin 83/35***

(45) Mention de la délivrance du brevet :
**22.01.86 Bulletin 86/04**

(84) Etats contractants désignés :
**AT BE CH DE FR GB IT LI LU NL SE**

(56) Documents cités :
**US-A- 3 657 265**
**CHEMICAL ABSTRACTS, vol. 94, no. 21, 25 mai 1981, page 675, no. 174516d, Columbus, Ohio, USA V.I. MANOV-YUVENSKII et al.: "Effect of catalyst composition and reaction conditions on the synthesis of phenyl isocyanate by the carbonylation of nitrobenzene"**
**CHEMICAL ABSTRACTS, vol. 87, no. 16, 17 octobre 1977, page 30, no. 118492m, Columbus, Ohio, USA B.K. NEFEDOV et al.: "Catalytic synthesis of 2,4-tolylene diisocyanate by the carbonylation of 2,4-dinitroteluene by carbon monoxide"**
**CHEMICAL ABSTRACTS, vol. 79, no. 11, 17 septembre 1973, page 449, no. 66345d, Columbus, Ohio, USA**

(73) Titulaire : **ATOCHEM**
**12/16, allée des Vosges**
**F-92400 Courbevoie (FR)**

(72) Inventeur : **Kervennal, Jacques**
**134, rue Docteur Locard**
**F-69005 Lyon (FR)**
Inventeur : **Cognion, Jean-Marie**
**85, route de Charly**
**F-69230 Saint-Genis Laval (FR)**
Inventeur : **Durual, Pierre**
**Les Essarts No. 2 Chemin du Rossignol**
**F-69390 Vernaison (FR)**

(74) Mandataire : **Rochet, Michel et al**
**ATOCHEM Département Propriété Industrielle Cedex 22**
**F-92091 Paris la Défense (FR)**

**EP 0 087 347 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un procédé de préparation de benzoxazolone-2 et dérivés par réaction, en présence d'une base hétéroaromatique azotée, d'orthonitrophénols avec du monoxyde de carbone sous pression élevée et en présence de catalyseurs supportés constitués d'un métal noble et d'un autre métal de transition déposés sous forme de particules ou d'oxydes métalliques.

La benzoxazolone-2 est un intermédiaire utilisé en chimie fine, notamment dans l'élaboration de certains colorants. La synthèse classique, à partir de l'orthonitrophénol nécessite deux étapes, la première, d'hydrogénation en orthoaminophénol, la deuxième, de phosgénation. Les inconvénients liés à l'utilisation du phosgène sont multiples : nécessité de synthétiser et de manipuler ce produit dangereux et sous-production de quantités importantes d'acide chlorhydrique qu'il faut, soit engager dans une réaction en consommant, soit électrolyser, ce qui exige l'implantation et l'entretien coûteux d'un atelier supplémentaire.

La mise au point d'un procédé permettant d'éviter l'utilisation du phosgène et de synthétiser la benzoxazolone-2 en une seule étape présente donc un réel intérêt.

Parmi les synthèses possibles, la réaction du monoxyde de carbone sur l'orthonitrophénol selon le schéma réactionnel suivant :

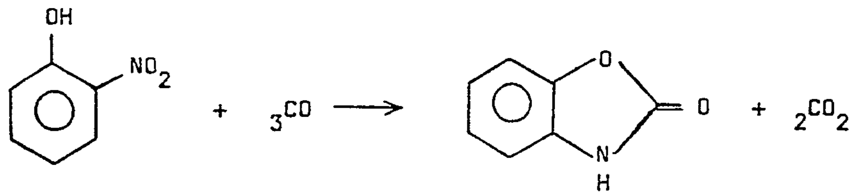

a déjà été décrite dans le brevet japonais 34 875/73. Cette synthèse est effectuée en présence d'halogénure d'un métal du groupe de la mine du platine associé à un oxyde métallique des groupes $V_b$, $VI_b$ ou VIII de la classification périodique, sans précision sur la récupération et le recyclage du catalyseur. Cependant, de tels systèmes dont la composition et la structure exacte sont mal définies, nécessitent des concentrations élevées en catalyseur pour obtenir des vitesses de réaction correctes.

La demanderesse a découvert que des systèmes catalytiques constitués d'un métal noble et d'un deuxième métal de transition présent sous forme de cristallites métalliques ou d'oxydes déposés sur support, associés à une base hétéroaromatique azotée s'avéraient particulièrement sélectifs et présentaient une activité accrue dans la carbonylation des orthonitrophénols en benzoxazolones-2.

Le procédé selon l'invention, est applicabe aux molécules d'orthonitrophénols de formule :

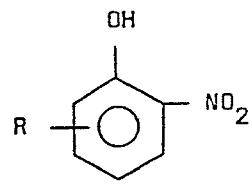

dans laquelle R représente un atome d'hydrogène ou d'halogène, un groupe alkyl contenant de 1 à 10 atomes de carbone, un groupe alkoxy OR' dans lequel R' représente un groupe alkyle ayant de 1 à 10 atomes de carbone.

La technique selon l'invention consiste à associer sur le support un métal noble du groupe VIII de la classification périodique à un deuxième métal de transition choisi dans les groupes $V_b$, $VI_b$ ou VIII et présent sous forme de particules métalliques ou d'oxydes.

Parmi les couples favorablement associés on peut citer, de façon non limitative, les systèmes suivants : palladium-molybdène, palladium-fer, palladium-vanadium, platine-molybdène, platine-fer.

Les métaux ou oxydes peuvent être déposés sur différents supports, à partir de divers composés minéraux ou organométalliques, selon des techniques connues d'imprégnation pour conduire, après traitement approprié, aux catalyseurs utilisés dans l'invention. Les composés métalliques peuvent être introduits l'un après l'autre mais il est préférable de les imprégner conjointement.

Ainsi, le support, sous atmosphère d'azote, peut être recouvert d'une solution des dérivés métalliques dans un solvant organique ou dans l'eau, puis l'ensemble est dégazé sous vide et le solvant distillé.

Selon un autre mode opératoire, on place le support dans un ballon d'évaporateur rotatif constamment et régulièrement agité et l'asperge d'une solution des dérivés métalliques, dans des conditions de température et de pression telles que le solvant est évaporé au fur et à mesure de son introduction.

Parmi les précurseurs métalliques qui peuvent être utilisés, on trouve notamment les acétates, nitrates, halogénures ou complexes organo-métalliques de métaux nobles ; les acétates, nitrates, halogénures, oxalates des autres métaux de transition, mais aussi des sels mixtes, tels que, par exemple, le molybdate d'ammonium, ou encore des complexes moléculaires hétéropolymétalliques associant directement les couples cités ci-dessus ; les clusters mixtes sont de bons exemples de telles espèces moléculaires.

Le support imprégné est, après séchage, porté à au moins 300 °C sous azote à l'aide d'une programmation linéaire de température, à raison de 2 °C/mn afin de décomposer les précurseurs en cristallites métalliques ou oxydes actifs dans la réaction étudiée.

Les supports pouvant être utilisés sont divers ; d'une façon non limitative, on peut citer les alumines, silices, silice-alumines, la magnésie, les charbons actifs, les carbures de silicium.

L'imprégnation peut être menée de façon à conduire à des teneurs en métal noble sur le support de 0,1 à 20 % en poids et préférentiellement de 1 à 15 %. Le rapport du nombre d'atomes-grammes du deuxième métal par rapport à celui du métal noble varie de $10^{-2}$ à $10^{2}$ et de préférence de 0,1 à 10.

Selon le procédé de l'invention, le nitrophénol est mis en contact avec l'oxyde de carbone à température et pression élevées en présence du catalyseur déposé sur support et de pyridine.

La présence de pyridine dans le milieu réactionnel permet d'améliorer l'activité et la sélectivité du catalyseur. Elle est ajoutée au milieu réactionnel dans des quantités allant de $10^{-2}$ à 30 moles par mole de dérivé nitré et de préférence de 0,1 à 10 moles par mole.

La concentration du catalyseur dans le milieu réactionnel, exprimée par le rapport du nombre d'atomes-grammes du métal noble sur le nombre de groupements nitrés à transformer varie de $10^{-4}$ à 1 et préférentiellement de $5.10^{-3}$ à $10^{-1}$.

La réaction peut être conduite en l'absence de solvant, en travaillant dans les o-nitrophénols fondus, mais la dilution dans un solvant peut favoriser la sélectivité. Les solvants utilisés de préférence sont les hydrocarbures saturés ou aromatiques tels que l'hexane, l'heptane, le n-décane, la décaline, le benzène, le toluène ou le xylène, les halogénures aromatiques tels que le chlorobenzène et les dichlorobenzènes ; ou des hétérocycles azotés comme la pyridine, la bipyridine ; on peut également utiliser des solvants fluorés tels que la méthyldécaline perfluorée, le trichlorotrifluoroéthane.

Quand on opère en présence de solvant, la proportion n'est pas critique, mais on travaille en général avec des solutions de 5 à 50 % en poids du dérivé nitré dans le solvant.

Les températures de réaction sont comprises entre 100 et 500 °C et plus particulièrement entre 150 °C et 250 °C.

Les pressions sont comprises entre 20 et 500 bars et de préférence entre 100 et 350 bars.

Il est possible d'opérer par une technique discontinue dans un appareillage du type autoclave ou par une technique continue qui permet d'éliminer dès sa formation la benzoxazolone produite.

Du fait de leur insolubilité dans le milieu réactionnel, les catalyseurs sont facilement récupérés après réaction par simple filtration et leur stabilité permet de les recycler sans perte d'activité.

Les essais décrits dans les exemples ci-dessous ont été réalisés en discontinu dans un autoclave en Hastelloy C de 500 ml, muni d'un dispositif d'agitation magnétique, pouvant opérer sous des pressions allant jusqu'à 500 bars et des températures de 300 °C. L'autoclave, chargé avec un orthonitrophénol, une base hétéroaromatique, le solvant éventuel et le catalyseur, est ensuite balayé à l'azote avant d'être mis sous pression d'oxyde de carbone à température ordinaire ; on le chauffe à la température choisie et l'avancement de la réaction est contrôlé par l'enregistrement de la pression. Après réaction, les teneurs en nitrophénol éventuellement résiduaire et en benzoxazolone formée ont été dosées par chromatographie liquide sous pression.

Les résultats indiqués s'entendent pour les définitions suivantes :

T.T.G., taux de transformation globale =

$$\frac{\text{nombre de moles de nitrophénol transformé}}{\text{nombre de moles de nitrophénol introduit}} \times 100$$

Sélectivité en benzoxazolone :

$$\frac{\text{nombre de moles de benzoxazolone formée}}{\text{nombre de moles de nitrophénol transformé}} \times 100$$

Rendement en benzoxazolone :

$$\frac{\text{nombre de moles de benzoxazolone formée}}{\text{nombre de moles de nitrophénol introduit}} \times 100 .$$

Exemple 1

Dans un ballon de 50 ml monté sur un évaporateur, on place 10 g d'une alumine pure commercialisée par CONDEA CHEMIE, de surface spécifique 250 m²/g et broyée en particules de 200 à 600 µm. On laisse dégazer le support pendant quinze minutes sous une pression partielle de 100 mm de mercure. On introduit ensuite, goutte à goutte, sur le support agité, 170 ml de solution aqueuse ammoniacale contenant 1,05 g d'acétate de palladium et 0,92 g de molybdate d'ammonium, en ajustant la température du bain d'huile de l'évaporateur de façon que la distillation du solvant soit instantanée. Après introduction de la totalité de la solution, le support imprégné et séché est transféré dans un tube de cuisson balayé à l'azote dont on élève progressivement la température à raison de 2 °C/mn jusqu'à 300 °C. Après un palier de 20 heures à cette température, on fait passer un courant d'hydrogène pendant 20 minutes puis refroidit sous azote. L'analyse indique une teneur de 4,8 % en palladium et de 5,8 % en molybdène.

On place, dans l'autoclave de 500 ml en Hastelloy C, 2,22 g du catalyseur précédemment préparé, 13,9 g d'orthonitrophénol (0,1 mole), 1 g de pyridine et complète à 100 ml le volume total à l'aide d'orthodichlorobenzène. Après balayage à l'azote, on comprime du monoxyde de carbone jusqu'à ce que la pression atteigne 200 bars à 20 °C. On isole l'autoclave et le chauffe, sous agitation, à 200 °C pendant 1 heure 40. On laisse refroidir et recueille le mélange réactionnel. Après récupération du catalyseur par filtration, le solvant est évaporé à sec et on obtient 13,5 g de résidu gris foncé dont le spectre infra-rouge correspond à celui de la benzoxazolone 2. L'analyse chromatographique montre que le T.T.G. du nitrophénol est de 100 % et la sélectivité en benzoxazolone-2 brute ($P^t$ fusion = 138 °C) de 100 %.

Après recristallisation dans l'eau chaude, le point de fusion est de 140 °C et l'analyse centésimale du produit est la suivante :

| | % C | % H | % N |
|---|---|---|---|
| - Théorique ........... | 62,22 | 3,70 | 10,37 |
| - Trouvé .............. | 62,02 | 3,71 | 10,11 |

Essais comparatifs :

ESSAI A — L'autoclave de 500 ml en Hastelloy C est chargé avec 13,9 g d'orthonitrophénol, 2,22 g du catalyseur précédemment préparé et le volume total est porté à 100 ml avec de l'orthodichlorobenzène. Après balayage à l'azote, on introduit du monoxyde de carbone jusqu'à une pression de 200 bars et la température est portée à 200 °C. Après 1 heure d'induction la pression baisse lentement et se stabilise au bout de 5 heures de réaction. Après refroidissement et distillation à sec du mélange réactionnel, celui-ci est analysé. Le T.T.G. est de 97 % et la sélectivité en benzoxazolone-2 brute de 91 %.

ESSAI B — L'autoclave en Hastelloy C est chargé avec 13,9 g d'orthonitrophénol, 2,1 g de catalyseur palladium à 5 % sur alumine commercialisé par la Société ENGELHARD (le support alumine a une surface spécifique supérieure à 100 m²/g), 1 g de pyridine, le volume étant complété à 100 ml par de l'orthodichlorobenzène. En opérant comme dans les exemples précédents, on amène la pression totale à 200 bars à température ordinaire à l'aide de monoxyde de carbone, puis chauffe à 200 °C. Après une heure d'induction sans consommation, on laisse réagir pendant 4 h 30. Le mélange réactionnel est ensuite analysé à la sortie du réacteur. Le T.T.G. est de 100 % et la sélectivité en benzoxazolone de 89,5 %.

Exemple 2

Selon la technique décrite dans l'exemple 1, on imprègne successivement sur 10 g de l'alumine pure commercialisée par CONDEA CHEMIE, 0,92 g de molybdate d'ammonium, puis 0,97 g d'acétate de palladium. Après le traitement thermique, on récupère un catalyseur dont les teneurs dosées en palladium et molybdène sont respectivement de 3,5 % et 4,5 %.

On introduit dans l'autoclave utilisé dans l'exemple 1, 13,9 g d'orthonitrophénol, 1 g de pyridine, 3 g du catalyseur et complète le volume total à 100 ml avec de l'orthodichlorobenzène. En ajustant la pression dans le réacteur à 200 bars à l'aide de monoxyde de carbone, on amène la température à 200 °C et on laisse réagir pendant 2 h 15. Après refroidissement et détente, on analyse le mélange. Le T.T.G. du nitrophénol est de 100 % et la sélectivité en benzoxazolone-2 de 91 %.

Exemple 3

13,9 g d'orthonitrophénol, 3 g du catalyseur préparé à l'exemple 2 et 1 g de pyridine sont introduits dans l'autoclave dont le volume de chargement est ajusté à 100 ml à l'aide d'orthodichlorobenzène. On compresse cette fois du monoxyde de carbone jusqu'à 100 bars, puis chauffe à 200 °C. On laisse réagir pendant 5 h 30, puis refroidit, détend et analyse le mélange réactionnel. Le T.T.G. est de 93,5 % et la sélectivité en benzoxazolone de 98 %.

Exemple 4

Un catalyseur mixte à base de palladium et de molybdène est préparé suivant le mode opératoire décrit dans l'exemple 1. Les teneurs respectives en palladium et molybdène, indiquées par l'analyse, sont de 4,7 % et 4,2 %.

On introduit dans l'autoclave 13,9 g d'orthonitrophénol, 2,3 g du catalyseur, 1 g de pyridine et complète le volume à 100 ml à l'aide d'orthodichlorobenzène. La pression est ajustée à 200 bars à froid avec du monoxyde de carbone, puis on chauffe à 200 °C. Après 1 h 50 de réaction, le réacteur est refroidi, vidé, le catalyseur récupéré par filtration et le mélange réactionnel analysé. Le T.T.G. est de 100 % et la sélectivité de benzoxazolone de 100 %.

Exemple 5

Les 2,3 g du catalyseur récupéré dans l'exemple précédent sont recyclés dans le réacteur avec 13,3 g d'orthonitrophénol, 1 g de pyridine et de l'orthodichlorobenzène comme solvant (q.s.p. 100 ml), de façon que le volume total soit de 100 ml. On opère dans les mêmes conditions que dans l'exemple 4, avec 200 bars de pression de monoxyde de carbone, et à 200 °C. On laisse également réagir 1 h 50, puis analyse le contenu du réacteur. Le T.T.G. de l'orthonitrophénol est de 100 % et la sélectivité en benzoxazolone-2 de 100 %. On ne constate donc pas de baisse d'activité ou de sélectivité au recyclage du catalyseur.

Exemple 6

Selon la technique décrite dans l'exemple 1, on imprègne conjointement sur 10 g de l'alumine pure commercialisée par CONDEA CHEMIE, une solution aqueuse contenant 1,05 g d'acétate de palladium et 2,07 g d'oxalate ferrique pentahydraté. Après le traitement thermique, on obtient un catalyseur dont les teneurs dosées en palladium et fer sont respectivement de 4,6 % et 4,0 %.

On introduit dans l'autoclave utilisé dans l'exemple 1, 13,9 g d'orthonitrophénol, 1 g de pyridine, 2,1 g du catalyseur et complète le volume à 100 ml avec de l'orthodichlorobenzène. En opérant comme dans les exemples précédents, on amène la pression totale à 200 bars à température ordinaire à l'aide de monoxyde de carbone, puis chauffe à 200 °C. On laisse réagir pendant 2 h 15 puis refroidit le réacteur et analyse le mélange. Le T.T.G. du nitrophénol est de 92 % et la sélectivité en benzoxazolone-2 de 95,6 %.

Exemple 7

On introduit dans l'autoclave de 500 ml, 15 g de chloro-4 nitro-2 phénol, 1 g de pyridine et 1,98 g du catalyseur de l'exemple 4. Le volume est complété à 100 ml à l'aide d'orthodichlorobenzène. La pression est ajustée à 200 bars à l'aide de monoxyde de carbone et on chauffe à 200 °C pendant 1 h 30. Après refroidissement on obtient, après filtration du catalyseur, 13,2 g de chloro-5 benzoxazolone-2 (Rdt 90 %).

Après recristallisation dans l'eau chaude, le point de fusion est de 188 °C et l'analyse centésimale du produit est la suivante :

| | % C | % H | % N | % Cl |
|---|---|---|---|---|
| - théorique ........ | 49,58 | 2,38 | 8,26 | 20,91 |
| - trouvé .......... | 50,67 | 2,34 | 8,23 | 20,92 |

Exemple 8

On imprègne 4 g d'alumine commerciale ayant une granulométrie comprise entre 200 et 600 µm et de surface spécifique 350 $m^2/g$ à l'aide d'une solution de 1 g de cluster mixte $Pd_2Cr_2(\eta^5-C_5H_5)_2(CO)_6(PPh_3)_2$ dans du chlorure de méthylène. Le cluster est préparé selon la méthode de P. Braunstein et al., Angew. Chem. Int. Ed. Engl, 1978, 17, 596 et R. Bender et P. Braunstein, J. Organometallic, chem., 172 (1979) $C_{51}$-$C_{54}$. Après traitement thermique à 350 °C, l'analyse indique que le catalyseur ainsi préparé contient 4 % de palladium et 1,9 % de chrome.

On place dans l'autoclave 6,9 g d'orthonitrophénol, 1,48 g du catalyseur et 0,7 g de pyridine et complète à 100 ml le volume total à l'aide d'orthodichlorobenzène. Après introduction de 200 bars de monoxyde de carbone, on chauffe le réacteur à 200 °C sous agitation pendant 3 heures. On laisse refroidir et analyse le mélange. Le T.T.G. de l'orthonitrophénol est de 28 % et la sélectivité en benzoxazolone-2 de 94 %.

**Revendications**

1. Procédé de préparation de benzoxazolone-2 et dérivés par réaction, en phase liquide, à une température de 100 à 500 °C et une pression de 20 à 500 bars, d'un orthonitrophénol de formule :

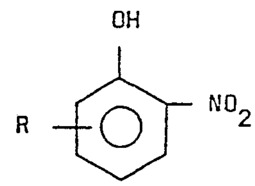

dans laquelle R représente un atome d'hydrogène ou d'halogène, un groupe alkyle contenant de 1 à 10 atomes de carbone, un groupe alkoxy OR' dans lequel R' représente un groupe alkyle contenant de 1 à 10 atomes de carbone,
et du monoxyde de carbone sous pression, caractérisé en ce que l'on opère en présence

a) d'un catalyseur supporté constitué par l'association sous forme métallique ou d'oxyde d'un métal noble du groupe VIII et d'un deuxième métal de transition choisi parmi les groupes Vb, VIb ou VIII de la classification périodique, et

b) de pyridine.

2. Procédé selon la revendication 1 dans lequel la concentration en poids du métal noble sur le support varie de 0,1 à 20 % en poids.

3. Procédé selon la revendication 1 ou 2 dans lequel le rapport atomique du deuxième métal sur le métal noble varie de $10^{-2}$ à $10^{2}$.

4. Procédé selon l'une des revendications 1 à 3 dans lequel la concentration du catalyseur dans le milieu réactionnel, exprimée par le rapport du nombre d'atomes grammes du métal noble sur le nombre de groupements nitrés à transformer varie de $10^{-4}$ à 1.

5. Procédé selon l'une des revendications 1 à 4 dans lequel la pyridine est introduite à des concentrations de $10^{-2}$ à 30 moles par mole de dérivé nitré.

6. Procédé selon l'une des revendications 1 à 5 dans lequel le métal noble est le palladium et le deuxième métal de transition le molybdène.

7. Procédé selon l'une des revendications 1 à 6 dans lequel on opère en présence d'un solvant.

8. Procédé selon la revendication 7 dans lequel le solvant est un hydrocarbure saturé ou aromatique ; un hologénure aromatique ; un hétérocycle azoté ; un solvant fluoré.

9. Procédé selon la revendication 7 ou 8 dans lequel on utilise des solutions contenant 5 à 50 % en poids du dérivé nitré dans le solvant.

10. Procédé selon l'une des revendications 1 à 8 dans lequel les supports du catalyseur sont les alumines, les silices, les silice-alumines, la magnésie, les charbons actifs ou les carbures de silicium.

**Claims**

1. Process for the preparation of benzoxazol-2-one and derivatives by liquid phase reaction, at a temperature of 100 to 500 °C and a pressure of 20 to 500 bars, of an ortho-nitrophenol of the formula :

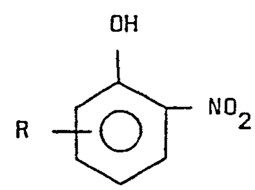

in which R represents a hydrogen or halogen atom, an alkyl group containing from 1 to 10 carbon atoms or an alkoxy group OR', in which R' represents an alkyl group containing from 1 to 10 carbon atoms, with carbon monoxide under pressure, characterised in that the reaction is carried out in the presence of

a) a supported catalyst consisting of the combination, in the metallic form or oxide form, of a noble metal of group VIII and a second transition metal chosen from among groups Vb, VIb or VIII of the Periodic Table and

b) pyridine.

2. Process according to Claim 1, in which the concentration by weight of the noble metal on the carrier varies from 0.1 to 20 % by weight.

3. Process according to Claim 1 or 2, in which the atomic ratio of the second metal to the noble metal varies from $10^{-2}$ to $10^{2}$.

4. Process according to one of Claims 1 to 3, in which the catalyst concentration in the reaction medium, expressed as the ratio of the number of gram atoms of noble metal to the number of nitro groups to be converted, varies from $10^{-4}$ to 1.

5. Process according to one of Claims 1 to 4, in which the pyridine is introduced at concentrations of $10^{-2}$ to 30 moles per mole of nitro derivative.

6. Process according to one of Claims 1 to 5, in which the noble metal is palladium and the second transition metal is molybdenum.

7. Process according to one of Claims 1 to 6, in which the reaction is carried out in the presence of a solvent.

8. Process according to Claim 7, in which the solvent is a saturated or aromatic hydrocarbon, an aromatic halide, a nitrogen-containing heterocyclic compound or a fluorine-containing solvent.

9. Process according to Claim 7 or 8, in which solutions containing 5 to 50 % by weight of the nitro derivative in the solvent are used.

10. Process according to one of Claims 1 to 8, in which the catalyst supports are aluminas, silicas, silicaaluminas, magnesia, active charcoals or silicon carbides.

**Patentansprüche**

1. Verfahren zur Herstellung von Benzoxazolon-2 und Derivaten durch Umsetzung eines Orthonitrophenols der Formel :

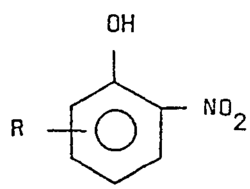

in der R ein Wasserstoff- oder Halogenatom, eine Alkylgruppe mit 1 bis 10 Kohlenstoffatomen, oder eine Alkoxygruppe OR′ bedeutet, in der R′ eine Alkylgruppe mit 1 bis 10 Kohlenstoffatomen ist, mit Kohlenmonoxid in der Flüssigphase bei einer Temperatur zwischen 100 und 500 °C und einem Druck von 20 bis 500 bar, dadurch gekennzeichnet, daß man arbeitet in Gegenwart von

a) einem Katalysator auf einem Träger, der durch Assoziation eines Edelmetalls der Gruppe VIII und eines zweiten Übergangsmetalls, das aus den Gruppen Vb, VIb oder VIII des Periodensystems ausgewählt ist, in metallischer Form oder in Form des Oxids gebildet wird, und

b) von Pyridin.

2. Verfahren nach Anspruch 1, bei dem die Konzentration des Edelmetalls auf dem Träger zwischen 0,1 und 20 Gew.% beträgt.

3. Verfahren nach Anspruch 1 oder 2, bei dem das Atomverhältnis des zweiten Metalls zu dem Edelmetall zwischen $10^{-2}$ und $10^{2}$ liegt.

4. Verfahren nach einem der Ansprüche 1 bis 3, bei dem die Konzentration des Katalysators im Reaktionsmedium, ausgedrückt durch das Verhältnis der Anzahl Gramm-Atom des Edelmetalls zu der Anzahl umzuwandelnder nitrierter Gruppen zwischen $10^{-4}$ und 1 liegt.

5. Verfahren nach einem der Ansprüche 1 bis 4, bei dem das Pyridin in Konzentrationen von $10^{-2}$ bis 30 Mol pro Mol Nitroderivat eingesetzt wird.

6. Verfahren nach einem der Ansprüche 1 bis 5, bei dem das Edelmetall Palladium und das zweite Übergangsmetall Molybdän ist.

7. Verfahren nach einem der Ansprüche 1 bis 6, bei dem man in Gegenwart eines Lösungsmittels arbeitet.

8. Verfahren nach Anspruch 7, bei dem das Lösungsmittel ein gesättigter oder aromatischer Kohlenwasserstoff, ein halogenierter Aromat, ein Stickstoffheterozyklus oder ein fluoriertes Lösungsmittel ist.

9. Verfahren nach Anspruch 7 oder 8, bei dem man Lösungen verwendet, die 5 bis 50 Gew.% des nitrierten Derivats in dem Lösungsmittel enthalten.

10. Verfahren nach einem der Ansprüche 1 bis 8, bei dem die Träger des Katalysators Aluminiumoxid, Siliciumoxide, Silico-Aluminiumoxide, Magnesiumoxid, Aktivkohlen oder Siliciumcarbide sind.